(19) Europäisches Patentamt
European Patent Office
Office européen des brevets

(11) Veröffentlichungsnummer: **0 099 517**
**B1**

(12) # EUROPÄISCHE PATENTSCHRIFT

(45) Veröffentlichungstag der Patentschrift: **26.11.86**

(51) Int. Cl.⁴: **C 12 P 1/00**

(21) Anmeldenummer: **83106608.9**

(22) Anmeldetag: **06.07.83**

(54) **Verfahren zur Durchführung elektromikrobieller Reduktionen.**

(30) Priorität: **17.07.82 DE 3226888**

(43) Veröffentlichungstag der Anmeldung: **01.02.84 Patentblatt 84/5**

(45) Bekanntmachung des Hinweises auf die Patenterteilung: **26.11.86 Patentblatt 86/48**

(84) Benannte Vertragsstaaten: **AT BE CH DE GB IT LI NL**

(56) Entgegenhaltungen:
**WO-A-80/00453**
**FR-A-2 495 843**

**ANGEWANDTE CHEMIE, INTERNATIONAL EDITION, Band 22, Nr. 4, 1983, Seiten 322-323, Verlag Chemie GmbH, Weinheim, DE; H. GÜNTHER u.a.: "Elecromicrobial reduction using yeasts"**
**ANGEWANDTE CHEMIE, INTERNATIONAL EDITION, Band 20, Nr. 10, 1981, Seiten 861-863, Verlag Chemie GmbH, Weinheim, DE; H. SIMON u.a.: "Electro-enzymatic and electro-microbial stereospecific reductions"**
**JOURNAL OF ORGANIC CHEMISTRY, Band 46, Nr. 22, 1981, Seiten 4622-4623, American Chemical Society, Easton, PA., US; R. DiCOSIMO u.a.: "Enzyme-catalyzed organic synthesis: electrochemical regeneration of NAD(P)H from NAD(P) using methyl viologen and flavoenzymes"**

(73) Patentinhaber: **BASF Aktiengesellschaft, Carl-Bosch- Strasse 38, D-6700 Ludwigshafen (DE)**

(72) Erfinder: **Simon, Helmut, Dr., Egilbertstrasse 31, D-8050 Freising (DE)**
Erfinder: **Bader, Johann, Dr., Am Hart 1b, D-8056 Neufahrn (DE)**
Erfinder: **Guenther, Helmut, Dr., Amperblick 11, D-8051 Haag (DE)**

(56) Entgegenhaltungen: (Fortsetzung)
**CHEMICAL ABSTRACTS, Band 88, Nr. 13, 13. Februar 1978, Seiten 214-215, Nr. 46811k, Columbus, Ohio, US; S.D. VARFOLOMEEV u.a.: "Bioelectrocatalysis. Hydrogenase as catalyst of electrochemical hydrogen ionization"**
**CHEMICAL ABSTRACTS, Band 88, Nr. 15, 10. April 1978, Seite 244, Nr. 101406j, Columbus, Ohio, US; G.V. DENISOV: "Possibility for the cultivation of chemolithotrophic microorganisms associated with the electrochemical reduction of the energy-producing substrate"**

**EP 0 099 517 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Durchführung elektromikrobieller Reduktionen mit Hilfe von Mikroorganismen, denen die Reduktionsäquivalente über einen Elektronenüberträger, der elektrochemisch regeneriert wird, zur Verfügung gestellt werden.

Es ist bereits bekannt, chemische Substanzen elektromikrobiell zu reduzieren. Diese Reduktionen erfolgen nach folgendem Schema:

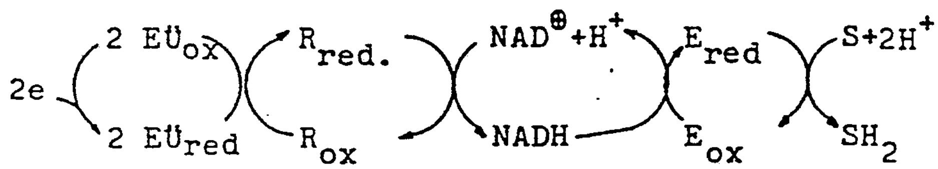

Elektronen e werden auf einen Elektronenüberträger $Eü_{ox}$ übertragen, der die Elektronen an eine Reduktase R weitergibt, welche die Reduktion von beispielsweise oxidiertem Nikotinamidadenindinukleotid ($NAD^{\oplus} \rightarrow NADH$) bewirkt (vgl. J. Elektroanal. Chem. 32, 415 (1971), J. Org. Chem. 46, 4623 (1981)). Dieses NADH kann als Reduktionsmittel zusammen mit einem zweiten Enzym E dienen, welches das gewünschte Substrat S reduziert. Die Kosten für die Enzymanreicherung sowie die Instabilität reiner Enzyme verhindern jedoch die Anwendung dieser Methoden für präparative Zwecke. Es wurde daher versucht, die Reduktion mit ganzen anaeroben Zellen, welche Reduktase enthalten, durchzuführen (Angew. Chem. 93, 897 (1981)). Auch dieses Verfahren ist für Synthesen in größerem Maßstab nicht viel besser, weil anaerobe Mikr oorganismen wegen ihrer Empfindlichkeit gegen Sauerstoff nur unter besonderem Aufwand zu züchten und einzusetzen sind.

Gegenstand der Erfindung ist ein Verfahren zur Durchführung elektromikrobieller Reduktionen mit Hilfe von Mikroorganismen, denen die Reduktionsäguivalente über einen Elektronenüberträger, der elektrochemisch regeneriert wird, zur Verfügung gestellt werden, dadurch gekennzeichnet, daß man als Mikroorganismen aerobe oder mikroaerophile Mikroorganismen verwendet und die Reduktion unter Ausschluß von Sauerstoff durchführt.

Die neue elektromikrobielle Reduktion läuft nach folgendem Schema ab:

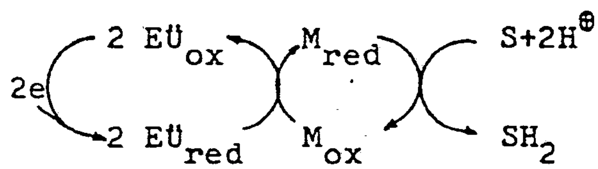

Die aus einer Stromquelle stammenden Elektronen e werden auf einen Elektronenüberträger $EÜ_{ox}$ übertragen, der die Elektronen an den aeroben Mikroorganismus $M_{ox}$ weitergibt. Dieser überträgt die Elektronen zusammen mit zwei Protonen auf das Substrat S, welches zu $SH_2$ reduziert wird.

Wenn die Zellen des aeroben Mikroorganismus eine Reduktase enthalten, welche von dem elektrochemisch regenerierten Elektronenüberträger EÜ reduziert werden kann, gelingt die Reduktion ganz glatt. Es existieren jedoch auch Mikroorganismen, die NADH- oder NADPH-abhängige Reduktasen enthalten. Unter diesen gibt es solche, die unter den Reaktionsbedingungen für NADH bzw. NADPH durchlässig werden und diese Verbindungen langsam verlieren. Es gibt weiter Mikroorganismen, die Enzyme enthalten, welche diese Pyridinnukleotide langsam zersetzen. In den letzten beiden Fällen empfiehlt es sich, die Reaktion durch Zugabe von geringen Mengen an Pyridinnukleotiden zum Reaktionsansatz zu beschleunigen.

Die Reduktion wird vorzugsweise in einer geteilten Zelle bei einer Temperatur von 5 bis 90°C, zweckmäßig 10 bis 50°C und vorzugsweise von 20 bis 40°C, und einem pH von 3 bis 10, vorzugsweise 5 bis 8, durchgeführt. Die Elektroden werden aus für die Elektrosynthese üblichem Elektrodenmaterial hergestellt. So sind beispielsweise Kathoden aus Metallen - wie Blei, Kupfer, Eisen, Nickel, Quecksilber, Stahl- oder Graphit-Halbleiter; mit Viologen-Farbstoffen dotiertes Nafion und Anoden aus Platin oder Graphit oder auch dimensionsstabile Anoden aus dotiertem oder beschichtetem Titan, wie sie zur Sauerstoff- oder Chlorentwicklung eingesetzt werden, geeignet. Als Trennwand zwischen Anolyt und Katholyt dienen handelsübliche Diaphragmen oder Membranen, vorzugsweise Ionenaustauschermembranen, wie sie beispielsweise zur Chloralkalielektrolyse oder zur Elektrodialyse Verwendung finden. Die Stromdichten betragen 1 bis 200, vorzugsweise 1 bis 100 $mA/cm^2$. Das Kathodenpotential liegt bei -0,1 bis -1,5 V, vorzugsweise zwischen -0,5 und -0,9 V, bezogen auf die Standardkalomel-Elektroden. Die Klemmenspannung der Zelle liegt zwischen 2 und 90 V, vorzugsweise 4 und 20 V.

Die Elektrolyse wird in der Regel in wäßrigen Gemischen durchgeführt, wobei die Mischungen neben dem

mikrobiellen System und dem Substrat auch Leitsalze, Puffer sowie organische Lösungsmittel oder Lösungsvermittler, z.B. Alkohole - wie Methanol oder Ethanol -, Ether - wie Dioxan, Dimethoxyethan oder Methyl-tert.butylether -, Emulgatoren - wie Polyoxyethylensorbitanmonoölsäureester -, Ester - wie Essigsäureethylester -, Alkane - wie Hexan, Petrolether -, chlorierte Kohlenwasserstoffe - wie Methylenchlorid, Tetrachlorkohlenstoff und Chloroform - oder Diemthylformamid enthalten können. Der Einsatz von organischen Lösungsmitteln ist möglich, insbesondere in Kombination mit immobilisierten Zellen. Beispiele sind gesättigte Alkohole, Dioxan, Furan, Dimethylsulfoxid etc. Weiter kann in Phasen gearbeitet werden, wobei die eine Phase ein Kohlenwasserstoff, Ether oder höherer Alkohol sein kann.

Die Verwendung von organischen Lösungsmitteln kann dann von Vorteil sein, wenn sich dadurch eine heterogene Reaktionsführung (z.B. fest/flüssig) vermeiden läßt. Entsteht bei der Reaktion ein in organischen Lösungsmitteln lösliches Produkt, welches den Mikroorganismus bzw. das darin enthaltene Enzym angreift, kann es angebracht sein, in 2 Phasen zu arbeiten.

Normalerweise besteht der Anolyt aus einer wäßrigen Salzlösung. Als Salze hierfür eignen sich beispielsweise NaCl, $Na_2SO_4$, $NaO-CO-CH_3$. Anstelle der Salzlösungen können auch verdünnte wäßrige Mineralsäuren verwendet werden. Der Katholyt besteht in der Regel ebenfalls aus einer Salzlösung, die zusätzlich das Substrat und den Mikroorganismus enthält. Vorteilhaft ist die Verwendung eines Puffers, wie Phosphatpuffer.

Als Elektronenüberträger kommen folgende Substanzen in Betracht:

1. Viologenfarbstoffe, z.B. Methylviologen, Benzylviologen, Diquat,
2. Anthrachinon und andere Chinon-Farbstoffe, z.B. Phenosafranin, Methylenblau, 2-Anthrachinonsulfonsäure,
3. Triphenylmethan-Farbstoffe, z.B. Methylviolett, Kristallviolett,
4. Phthalocyanine, z.B. Fe-, Cu- oder Co-, Phthalocyanin,
5. Methinfarbstoffe, z.B. Astraphloxin,
6. Pyrrolfarbstoffe oder Porphyrinderivate, z.B. Metall-Chelatkomplexe dieser Verbindungen,
7. Pteridine und Pteridone,
8. Flavine, z.B. Acriflavin, Lumiflavin,
9. Imidazol-Derivate, z.B. Metronidazol,
10. Metallkomplexe der Metalle der 6., 7., 8. Nebengruppe z.B. $Ru(L_2L'_2)^{++}$ [L=1,10 Phenanthrolin, 2,2-Bipyridyl oder 5-Nitro-1,10-phenanrolin; L'=Pyridin oder 4-Methylpyridin], 1,1'-Bis(hydroxymethyl)ferrocene bzw. Ferrocen-Monocarbonsäuren,
11. Thiolate mit den Metallen der 6., 7. oder 8. Nebengruppe
12. Thiole, z.B. Dihydroliponsäure, Dithiothreitol, 2-Mer-captoethanol, Glutathion, Thiophenol, 1,4-Butandithiol,
13. $NAD^+$- bzw. $NADP^+$ oder deren Derivate.

Unter diesen ist die 1. Gruppe, insbesondere das Methylund Benzylviologen bevorzugt.

Für die Reduktionsreaktion eignen sich alle aeroben Mikroorganismen, sofern sie die für die gewünschte Umsetzung erforderlichen Enzyme enthalten. Wichtige Beispiele für Mikroorganismen sind:

A) Prokaryonten:

Gram-negative aerobe Bakterien, z.B. Acetobacter ascendens, Acetobacter pasteurianus, Alcaligenes eutrophus, Pseudomonas aeruginosa, Pseudomonas fluorescens, Pseudomonas testosteroni; Gram-negative fakultativ anaerobe Bakterien, z.B. Enterobacter aerogenes, Enterobacter agglomerans, Escherichia coli, Flavobacterium spec., Proteus mirabilis, Proteus vulgaris, Proteus mitajiri, Zymomonas mobilis; Gram-positive Kokken, z.B. Leuconostoc mesenteroides, Peptococcus aerogenes, Sarcina lutea, Streptococcus faecalis; Endosporen-bildende Bakterien, z.B. Bacillus subtilis, Bacillus cereus, Bacillus polymyxa; Gram-positive, asporogene Bakterien, z.B. Lactobacillus buchneri; Coryneforme Bakterien, z.B. Arthrobacter spec., Corynebacterium simplex; Actinomyceten, z.B. Actinomyces globosus, Mycobacterium spec., Nocardia corallina, Streptomyces platensis, Streptomyces lavendulae;

B) Eukaryonten:.

Phycomyceten, z.B. Absidia orchidis, Rhizopus arrhizus, Rhizopus nigricans, Rhizopus reflexus; Protoascomyceten (Hefen), z.B. Candida pseudotropicalis, Geotrichum candidum, Hansenula capsulata, Kloeckera magna, Kluyveromyces fragilis, Rhodotorula mucilaginosa, Rhodotorula glutinis, Saccharomyces cerevisiae, Saccharomyces sake, Saccharomyces fragilis, Saccharomyces uvarum, Schizosaccharomyces pombe, Candida utilis, Candida boidenii; Ascomyceten, z.B. Aspergilius niger, Aspergillus nidulans, Cladosporium butyri, Claviceps spec., Dipodascus albidus, Eremothecium spec., Penicillium chrysogenum; Fungi imperfecti, z.B. Curvularia falcata, Epicoccum oryzae, Fusarium lateritium, Fusarium solani, Phialophora spec.

Weiterhin sind als Mikroorganismen aerobe Protozoen sowie aerobe Zellen von höheren Pflanzen und Tieren zu verstehen, soweit sich diese wie Mikroorganismen züchten lassen. Solche Mikroorganismen können beispielsweise von Stammsammlungen bezogen oder selbst gezüchtet werden.

Von den genannten Mikroorganismen sind Proteus mirabilis, Proteus vulgaris, Alcaligenes eutrophus, Bacillus cereus, Geotrichum candidum, Kloeckera magna, Saccharomyces cerevisiae und Candida utilis besonders bevorzugt.

Es gibt Fälle, in denen ein Mikroorganismus besonders effektiv NADH oder NADPH bildet, während die Reduktase zur Umsetzung des Substrates S in einem anderen Mikroorganismus eine hohe Aktivität aufweist,

der jedoch nur eine geringe Aktivität für die NADH- bzw. NADHP-Bildung besitzt. In diesen Fällen empfiehlt es sich, mit einem Gemisch der beiden Mikroorganismen zu arbeiten.

Die Mikroorganismen bzw. Zellen können auch in immobilisierter Form für die Umsetzungen verwendet werden. Weiter kann die Permeabilität der Mikroorganismen für Cosubstrate, Substrate und Produkte in einer Reihe von Fällen, beispielsweise durch Einfrieren und Auftauen der Zellen erhöht werden.

Obwohl die Reduktion mit aeroben Zellen durchgeführt wird, darf praktisch kein Sauerstoff bei der Reaktion zugegen sein. Der Sauerstoffgehalt muß so gering sein, daß eine eventuell mögliche Reaktion des Sauerstoffs mit dem Elektronenüberträger keine Rolle spielt und daß keine inhibitorische Wirkung des Sauerstoffs oder eines durch die Anwesenheit von Sauerstoff entstehenden Oxidationsproduktes auf die bei der Reaktion anwesenden Enzyme und Cosubstrate auftritt. Steigt der Sauerstoffgehalt im Katholyten z.B. bei Verwendung von Methylviologen auf über $5.10^{-7}$ M an, so sinkt die Stromausbeute und die Stabilität der Biokatalysatoren mit steigendem Sauerstoffgehalt zunehmend ab.

Weitere Beispiele für elektromikrobielle Reduktionen gemäß der Erfindung sind:

I. Reduktionen von Carbonylgruppen

1. Selektive Reduktion von Aldehydgruppen in Anwesenheit anderer reduzierbarer Funktionen

$$^{1}R^{2}RC{=}C^{3}RCHO \longrightarrow {}^{1}R^{2}RC{=}C^{3}RCH_2OH$$

z.B. Darstellung von 2-substituierten Zimtalkoholen aus entsprechenden Zimtaldehyden, oder Hydroxyacetophenon aus Phenylglyoxal, und anderen Hydroxyketonen aus Diketonen.

2. Herstellung primärer Alkohole, die durch stereospezifischen Ersatz eines Wasserstoffatoms durch ein Deuterium- oder Tritiumatom chiral sind, durch Reduktionen in Deuteriumoxid oder in Tritium-markiertem Wasser.

3. Selektive Reduktionen von Ketogruppen in Anwesenheit anderer reduzierbarer Funktionen. Als Beispiele seien genannt:

$C_6H_5CH{=}CRCOCH_3 \rightarrow C_6H_5CH{=}CRCHOHCH_3$

oder

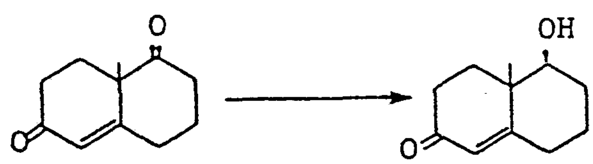

oder

$\Delta^4$-Androsten-3,17-dion zu Testosteron

4. Gewinnung chiraler cyclischer und nicht-cyclischer Alkohole, Hydroxysäuren und dgl.:

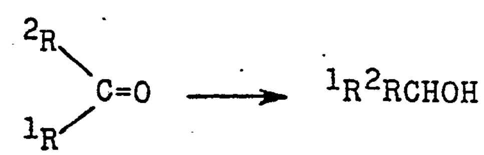

z.B. (R)-Mandelsäure aus Phenylglyoxylsäure oder (R)-Phenyllactat aus Phenylpyruvat. Bei der Reduktion von entsprechend substituierten Ketonen oder Cycloketonen kann es bei Substrat- und Produktspezifität des Enzymsystems gleichzeitig zu einer Racemattrennung kommen.

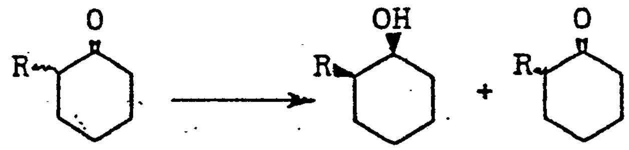

Je nach Spezifität des Enzymsystems kann es zu einem von drei weiteren Paaren kommen.

II. Selektive Reduktion von ungesättigten Gruppen in prochiralen oder achiralen Molekülen

1. Selektive Reduktionen in Gegenwart von anderen reduzierbaren Gruppen, z.B. Reduktionen von Sorbinsäure zu $\Delta^4$-Pentencarbonsäure, oder α,β-ungesättigten Aldehyden zu gesättigten Aldehyden.

2. Reduktionen von α,β-ungesättigten Carbonyl- und Carboxylverbindungen, insbesondere solche, die durch entsprechende Substitution zu chiralen Produkten führen

$R^1R^2C{=}CXY \rightarrow R^1R^2CHCHXY$

Y= $COO^-$, CHO, COR; X= H, Alkyl, Alkoxy, Alkthio, Halogen, Dialkyl- oder Arylamino; $R^1$ und $R^2$ = H, Alkyl, Alkoxy, Aryl, Alkoxycarbonyl, Alkenyl.

Als Beispiele für solche Hydrierungsprodukte seien insbesondere genannt: chirale Halogencarbonsäuren, wie z.B. 3-(p-Chlorphenyl)-2-chlorpropion-säure; chirale α oder β-Alkyl-verzweigte Carbonsäuren wie (R)- oder (S)-2-Methyl-3-phenylpropionsäure , 2-Amino-3-methyl-3-phenylpropionsäure, oder $\Delta^3$-2- und/oder 4-substituierte Carbonsäuren aus den entsprechenden Allencarbonsäuren. Dabei können, ausgehend von einem Racemat (Molekülasymmetrie:) chirale E/Z Isomere gebildet werden, die sich leicht trennen lassen.

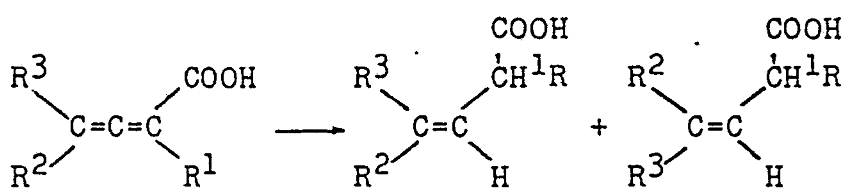

Beispiele für die Reduktion von Aldehyden zu chiralen Produkten sind die Gewinnung von (R)- oder (S)-Citronellal oder Citronellol aus cis- oder trans-Citral.

3. Reduktionen isolierter C=C-Doppelbindungen, die bei gegebener Substitution auch zu chiralen Verbindungen führen können.

4. Reduktionen von C=C-Doppelbindungen in markiertem Wasser, um zu Verbindungen zu kommen, die durch stereospezifischen Ersatz von H durch $^2H$ oder $^3H$ in einer Methylen- oder Methylgruppe chiral werden, z.B. [2,3-2H] Dideuterobutyrat oder [2 3-2H]-Dideuterophenylpropionat. Eine chirale Methylgruppe kann durch Reduktion von (E)- oder (Z)-$CH^3H{=}CHCOOH$ in $^2H_2O$ erhalten werden.

III. Reduktive Aminierung von Carbonylverbindungen, insbesondere von Ketosäuren zu Aminosäuren, wie z.B. Umsetzung von 2-Oxo-5-methylpentancarbon-säure zu (S)-Leucin.

Die Isolierung der Endprodukte aus den Reaktionslösungen erfolgt in an sich bekannter Weise, beispielsweise durch Destillation, Extraktion, Kristallisation oder Chromatographie.

Das neue Verfahren hat gegenüber dem Verfahren, welches mit anaeroben Mikroorganismen durchgeführt wird, folgende Vorteile.

Die Verwendung und der Einsatz aerober Mikroorganismen ist wegen ihrer Unempfindlichkeit gegen Sauerstoff viel einfacher.

Aerobe Mikroorganismen lassen sich einfacher als anaerobe herstellen. Außerdem werden mit ihnen wesentlich höhere Zelldichten erreicht, so daß der apparative Aufwand zu ihrer Herstellung geringer ist.

Weiter war nicht vorhersehbar, daß man mit aeroben Mikroorganismen Reaktionen durchführen kann, ohne Sauerstoff in das Reaktionsmedium einzuleiten, weil Umsetzungen mit aeroben Mikroorganismen in aller Regel nur bei starker Belüftung gelingen.

Dem Reaktionsmedium brauchen keine Kohlenhydrate zugesetzt zu werden. Dadurch entstehen keine Nebenprodukte, die abgetrennt werden müssen.

Die folgenden Beispiele erläutern die Erfindung. In allen Beispielen wurde während der elektromikrobiellen Reduktion unter Ausschluß von Luftsauerstoff gearbeitet. Die verwendete elektrolytische Zelle ist in Angew. Chem. 93, 897 (1981) beschrieben.

**Beispiel 1**

Herstellung von (2R)-Propandiol

50 µmol Methylviologen, 2,5 mMol Kaliumphosphat und 400 mg Candida utilis (z.B. DSM 70 167) wurden in 25 ml Wasser gelöst bzw. aufgeschlämmt und der pH-Wert auf 7,0 justiert. Das so erhaltene Gemisch wurde in eine elektrochemische Zelle gegeben und bei einem konstanten Kathodenpotential von -790 mV gegenüber der Standardkalomelelektrode (SCE) das Methylviologen reduziert. Dabei ergab sich ein Nullstrom von etwa 0,25 mA. Anschliessend wurde 1 mMol Acetol zugegeben und das Potential von -79( mV aufrechterhalten. Nach 50 h war das Acetol quantitativ in (2R)-Pro-pandiol überführt, welches nach Zentrifugation destillativ abgetrennt wurde. Das (2R)-Propandiol besaß den Drehwert $[\alpha]^{20}_D = -20,7°$

**Beispiel 2**

Herstellung von (2R)-Propandiol

Für dieses Beispiel wurde das in Beispiel 1 verwendete Ausgangsgemisch, jedoch nach Zugabe von 19µmol NAD⊕ verwendet. Zur Lösung mit dem reduzierten Methylviologen wurden 4 mMol Acetol gegeben und die Spannung bei -790 mV aufrechterhalten. Nach 22 h wurden noch einmal 19µMol NAD⊕ zugegeben, um den Strom, der inzwischen auf etwa 2,5 mA abgefallen war, wieder zu erhöhen. Nach 45 h war das Acetol vollständig zu (2R)-Propandiol umgesetzt, welches analog Beispiel 1 isoliert und charakterisiert wurde, $[\alpha]_D^{20} = -20,5°$.

**Beispiel 3**

Herstellung von (R)-Pantolacton

Analog Beispiel 2 wurden 250µmol Natrium-2-ketopantoat reduziert. Es wurden jedoch 6,4µmol-NAD⊕ zugesetzt und diese Zugabe nach 30 und 50 h wiederholt. Nach 70 h war die Umsetzung quantitativ. Das nach Ansäuern gebildete (R)-Pantolacton wurde mit Ether extrahiert und sublimiert. Es war in Gegenwart eines chiralen Shiftreagens NMR-spektroskopisch vermessen mehr als 99,5 % optisch rein.

Dieselbe Reaktion ließ sich auch mit Benzylviologen bei einem Kathodenpotential von -620 mV gegenüber der SCE durchführen.

Weiter ließ sich die Reduktion auch mit Proteus mirabilis (DSM 30 115) sowie Proteus vulgaris (DSM 30 118) durchführen. Dabei ließen sich Ströme von 15 mA pro 20 mg Zellmaterial erhalten.

**Beispiel 4**

Herstellung von (R)-Methylbernsteinsäure

180µmol Methylviologen, 9 mmol Kaliumphosphat, 9µmol EDTA und 1,11 g E.coli (K 12, beziehbar von Deutsche Sammlung von Mikroorganismen, Göttingen) wurden in wenig Wasser gelöst bzw. aufgeschlämmt. Nach Einstellen des pH-Wertes auf 7,0 wurde mit Wasser auf 90 ml aufgefüllt und 1,6 mMol Mesaconat zugegeben. Das so erhaltene Gemisch wurde analog Beispiel 1 bei -790 mV reduziert. Nach 42,5 h waren 98,5 % (R)-Methylbernsteinsäure entstanden, welche durch Etherextraktion der angesäuerten Lösung isoliert wurde. Sie besaß den Drehwert $[\alpha]_D^{20} = +9,2°$.

**Beispiel 5**

Herstellung von Propandiol

160µmol Methylviologen, 4 mmol Tris(hydroxymethyl)-aminomethanacetat wurden in 40 ml Wasser gelöst und der pH-Wert auf 7,0 gebracht. Diese Lösung wurde in einer elektrochemischen Zelle bei einem konstanten Kathodenpotential von -700 mV reduziert. Anschließend wurden 0,5 ml einer Suspension von Bacillus cereus (DSM 31) entsprechend einer Trockenmasse von 10 mg, 32µmol NAD, 1200µmol Acetol sowie 0,3 ml einer Suspension von Alcaligenes eutrophus H 16 (DSM 428) entsprechend einer Trockenmasse von 2,0 mg zugegeben. Im kompletten System floß ein Strom von 4,5 mA. Das eingesetzte Acetol wurde in 18 h zu über 90 % reduziert.

Die gleiche Umsetzung ließ sich durch die Kombination von Candida utilis und Alcaligenes eutrophus erreichen. Die erzielte Reduktionsgeschwindigkeit pro Gewichtseinheit Biokatalysator (Summe beider Organismen) ist dabei etwa 10 mal höher als in Beispiel 1.

**Beispiel 6**

Reduktion von Phenylpyruvat oder 2-Oxo-4-methylpentanat zu den entsprechenden 2-Hydroxysäuren

Analog Beispiel 5 wurden die Rohlysate der beiden dort angegebenen Mikroorganismen und die anderen Komponenten, sowie Phenylpyruvat bzw. 2-Oxo-4-methylpentanat in einer elektrochemischen Zelle umgesetzt. Die Reduktion lief mit einem Strom von etwa 0,5 mA.

**Patentansprüche**

Verfahren zur Durchführung elektromikrobieller Reduktionen mit Hilfe von Mikroorganismen denen die Reduktionsäquivalente über einen Elektronenüberträger der elektrochemisch regeneriert wird, zur Verfügung gestellt werden, dadurch gekennzeichnet, daß man als Mikroorganismen aerobe oder mikroaerophile Mikroorganismen einzeln oder in Kombination miteinander verwendet und die Reduktion unter Ausschluß von Sauerstoff durchführt.

**Claim**

A method of carrying out an electromicrobial reduction with the aid of microorganisms which receive the reduction equivalents via an electron carrier which is regenerated electrochemically, wherein aerobic or microaerophilic microorganisms, either alone or in combination with one anotter, are used as the microorganisms, and the reduction is carried out in the absence of oxygen.

**Revendication**

Procédé pour la réalisation de réductions électromicrobiennes à l'aide de micro-organismes, auxquels les équivalents de réduction sont fournis par un agent de transfert d'electrons, resgénéré par voie électrochimique, caractérisé en ce que les micro-organismes sont choisis parmi les micro-organismes aérobies et micro-aérophiles, utilisés seuls ou combinés entre eux, et la réduction et réalisée à l'abri de l'oxygène.